# FASCICULE DE BREVET EUROPEEN

(11) **EP 1 404 631 B1**
(45) Date de publication et mention de la délivrance du brevet: **02.11.2005**
(21) Numéro de dépôt: 02764946.6
(22) Date de dépôt: 05.07.2002
(51) Int. Cl.: C07B 47/00, B01J 31/16, G01N 33/547, G01N 33/543

(54) **PROCEDE DE FONCTIONNALISATION DE SUPPORTS SOLIDES**
VERFAHREN ZUR FUNKTIONALISIERUNG VON FESTEN TRÄGERN
METHOD OF FUNCTIONALISING SOLID SUPPORTS

(30) Priorité: 09.07.2001 FR 0109082
(43) Date de publication de la demande: 07.04.2004
(73) Titulaire: CENTRE NATIONAL DE LA RECHERCHE SCIENTIFIQUE (CNRS), 75016 Paris (FR); ECOLE CENTRALE DE LYON, 69130 Ecully (FR)
(72) Inventeur: DUGAS, Vincent, F-69330 Meyzieu (FR); CHEVALIER, Yves, F-69540 Irigny (FR); SOUTEYRAND, Eliane, F-69130 Ecully (FR)
(74) Mandataire: Goulard, Sophie
(86) Numéro de dépôt international: PCT/FR2002/002364
(87) Numéro de publication internationale: WO 2003/008360

(56) Documents cités:
- US-A- 5 306 853
- US-A- 5 565 592
- US-B1- 6 235 488
- ANNIS, D.A.; JACOBSEN, E.N.: "Polymer Supported Chiral Co(Salen) Complexes: SyntheticApplications and Mechanistic Investigations in the Hydrolytic Kinetic Resolution of Terminal Epoxides" J. AM. CHEM. SOC., vol. 121, 1999, pages 4147-54, XP001019176
- LOSSET ET AL.: "Induction asymétrique supramoléculaire par un réactif modèle du NADH greffé sur silice" BULL. SOC. CHIM. FR., vol. 128, 1991, pages 721-9, XP002196786

## Description

La présente invention se rapporte à un procédé de fonctionnalisation de supports solides, aux supports solides fonctionnalisés obtenus en mettant en oeuvre un tel procédé, à leurs utilisations, en particulier pour l'immobilisation de molécules biologiques, telles que des acides nucléiques, des polypeptides, des lipides, des carbohydrates et des hormones.

Les supports porteurs de molécules biologiques immobilisées sont avantageusement utilisés pour la détection et la reconnaissance d'espèces biologiques, mais d'autres applications de ces supports, comme la synthèse ou modification chimique sur support ou encore l'exaltation de réactions biologiques sur support, sont également possibles.

Dans le cadre particulier de la détection et de la reconnaissance d'espèces biologiques, il est primordial de pouvoir disposer de supports solides fonctionnalisés présentant un certain nombre de qualités.

Ces supports doivent en particulier permettre l'immobilisation reproductible des molécules biologiques d'intérêt, dans la mesure où une immobilisation reproductible est conditionnelle d'une détection elle-même reproductible.

Ces supports doivent également permettre l'immobilisation des molécules biologiques d'intérêt de façon sensible. La sensibilité d'un support solide fonctionnalisé dépend du taux d'immobilisation et de la méthode de détection d'un signal, mais aussi et surtout du niveau du bruit de fond (signal non spécifique). Une diminution du bruit de fond améliore le rapport signal/bruit. En effet, dans un dispositif dans lequel on détecte la présence d'espèces biologiques au voisinage de la surface, le bruit de fond vient essentiellement de l'absorption non spécifique de molécules autres que les molécules biologiques d'intérêt que l'on souhaite immobiliser et qu'il convient par conséquent de limiter.

D'autre part, il est important de pouvoir disposer de supports solides fonctionnalisés réutilisables. En effet, la plupart des supports fonctionnalisés actuellement disponibles sont qualifiés de jetables, ce qui peut, bien évidemment, être considéré comme un avantage par l'utilisateur. Cependant, ces supports sont jetables car il n'est tout simplement pas possible de les utiliser plusieurs fois, notamment à cause de la forte dérive du signal mesuré. Le qualificatif "jetable" devient alors synonyme de "qualité médiocre". La possibilité de pouvoir disposer d'un support réutilisable est d'un grand intérêt car elle autorise l'analyse de plusieurs échantillons biologiques avec un seul et même dispositif, ce qui permet d'effectuer des comparaisons quantitatives. De plus, les supports réutilisables autorisent la réalisation de plusieurs mesures sur un même échantillon et permettent ainsi l'amélioration des résultats d'un point de vue statistique, ce qui n'est pas envisageable avec un support jetable qui dérive trop rapidement en raison de la mauvaise immobilisation des molécules biologiques sur le support.

Enfin, sur un support fonctionnalisé sur lequel on souhaiterait localiser de façon précise les espèces immobilisées sur des plots, l'étape d'immobilisation doit être réalisée de manière bien localisée et homogène sur la surface des plots, afin d'éviter tout risque de bavures ou d'auréoles.

L'immobilisation de molécules biologiques sur des supports solides s'effectue généralement en deux étapes distinctes :
- une première étape de fonctionnalisation des supports qui consiste en une modification chimique de leur surface par greffage de molécules de synthèse (agents de couplage) qui vont assurer la fixation des molécules biologiques sur le support ;
- une deuxième étape d'immobilisation consistant à établir une liaison covalente entre les molécules biologiques et ces supports fonctionnalisés.

Afin d'établir de telles liaisons covalentes, différents types de réactions peuvent être établies en fonction de la nature, d'une part de l'agent de couplage et de la molécule biologique à immobiliser d'autre part.

Ainsi, en ce qui concerne la fixation des protéines ou des oligonucléotides (porteurs d'un bras espaceur aminé), la réaction avec la surface fait intervenir des fonctions amines. La fixation en surface de telles fonctions est assurée principalement par des liaisons de type thiourée ou amide. La formation des thiourées a en particulier été décrite dans l'article de Guo Z. *et al*., Nucleic Acid Research, 1994, **22**, 5456-5465 et consiste à activer des surfaces aminées par des molécules soufrées telles que par exemple le phénylènediisothiocyanate, puis à faire réagir les molécules sur la surface ainsi activée. La formation des liaisons amides est obtenue par réactions d'aminés sur des acides carboxyliques en général par modification chimique d'un support aminé (par exemple par l'anhydride succinique ou une réaction de polymérisation) comme cela est décrit par exemple dans les brevets américains n° 5 919 523, 5 667 976 et 6 043 353.

La fonctionnalisation directe sur support solide par des acides carboxyliques peut être réalisée par la formation de couches auto-assemblées d'alkanethiols acides sur des dépôts d'or (BONCHEVA M. *et al*., Langmuir, 1999, **15,** 4317-4320 ; HUANG E. *et al*., Langmuir, 2000, **16**, 3272-3280) ou bien encore par des réactions d'hydrosilylation sur des supports de silicium (Strother T. *et al*., J. Am. Chem. Soc., 2000, **122,** 1205-1209) ; cependant, dans ce dernier cas, la fixation des biomolécules implique des réactions de complexation.

Dans le cas particulier de l'immobilisation de protéines, il a déjà été proposé de fonctionnaliser des supports en quartz par un silane porteur de groupements ester qui sont ensuite déprotégés (Brennan J. D. *et al*., Can. J. Chem., 1994, 72, 721-728).

La modification chimique des molécules biologiques a également permis de développer d'autres méthodes d'immobilisation. Ainsi, il a été proposé une méthode d'immobilisation électrochimique basée sur la polymérisation du pyrrole (Livache T. *et al*., Biosensors & Bioelectronics, 1998, **13**, 629-634 et Garnier F. *et al*., Synthetic Metals, 1999, **100,** 89-94). Cette méthode permet l'immobilisation de molécules (oligonucléotides, peptides) porteuses de résidus pyrrolyle par un accrochage localisé, mais impose l'utilisation de supports conducteurs et nécessite une méthode d'adressage multiplexée.

Il a également été proposé de modifier des oligonucléotides par un groupement dialdéhyde afin de permettre leur fixation sur des fonctions hydrazides. (Proudnikov D. *et al*., Analytical Biochemistry, 1998, **259,** 34-41 et Dubiley S. *et al*., Nucleic Acid Research, 1997, **25**, 2259-2265). Les supports fonctionnalisés sont, dans ce cas, des gels de polyacrylamide ou de nitrocellulose qui forment des réseaux épais et donc tridimensionnels qui ont l'avantage d'augmenter, pour une même surface, la densité des brins immobilisés. Ces gels présentent néanmoins quelques limites concernant la taille des plots déposés et la taille des brins d'ADN qui peuvent être hybridés (Yershov G. *et al*., Proc. Natl. Acad. Sci. USA, 1996, **93**, 4913-4918).

Enfin, une voie de plus en plus utilisée, implique la réaction d'oligonucléotides porteurs d'un bras espaceur terminé par une fonction thiol sur des surfaces fonctionnalisées par des haloacétates (US 5 412 087 et Tang K. *et al*., Proc. Natl. Acad. Sci. USA, 1999, **96,** 10016-10020). L'établissement de ponts disulfure ou de liaisons thioéther, après addition d'un groupement pyridylsulfure sur l'oligonucléotide (WO 99/20640), est possible sur des surfaces fonctionnalisées par des thiols. Si les thiols semblent assez réactifs et insensibles à l'humidité, ces fonctions sont néanmoins délicates d'emploi car elles ont tendance à s'oxyder en disulfure. Une réduction préalable du disulfure in situ est donc souvent nécessaire pour espérer obtenir des résultats reproductibles.

Il existe donc par conséquent un large éventail de possibilités pour aboutir à des supports fonctionnalisés permettant l'immobilisation de molécules biologiques d'intérêt. Cependant, à ce jour, l'obtention de supports solides fonctionnalisés réutilisables permettant l'immobilisation de molécules biologiques d'intérêt de façon reproductible et sensible, ainsi que la détection d'un signal en limitant le rapport signal/bruit n'a pu être réalisée de façon totalement satisfaisante.

C'est pourquoi les Inventeurs se sont donnés pur but de pourvoir à de tels supports et ont mis au point ce qui fait l'objet de la présente Invention.

La présente invention a pour premier objet un procédé de fonctionnalisation d'un support solide comportant en surface des fonctions hydroxyle ou hydrure, comprenant les étapes suivantes :
a) le greffage par fixation covalente (silylation ou hydrosilylation) d'au moins une molécule bifonctionnelle comportant une fonction acide carboxylique protégée de formule (I) suivante :

   A-X-COOR (I)

   dans laquelle :
   - A représente un groupement permettant la fixation covalente de la molécule bifonctionnelle de formule (I) sur les fonctions hydroxyle ou hydrure du support,
   - R représente un groupement protecteur de la fonction acide carboxylique,
   - X représente une chaîne hydrocarbonée en C₂-C₁₈, linéaire ou ramifiée, saturée ou insaturée, éventuellement interrompue par un ou plusieurs hétéroatomes choisis parmi N, O et S ;
b) la déprotection des fonctions acide carboxylique n'ayant pas été déprotégées au cours de l'étape a) de greffage ;
caractérisé en ce que l'étape a) est réalisée à une température comprise entre 50 et 200°C avec un composé de formule (I) dans lequel A est un groupement monofonctionnel et en ce que ledit procédé comprend en outre une étape c) de passivation des fonctions hydroxyle ou hydrure résiduelles du support n'ayant pas réagi avec ladite ou lesdites molécules de formule (I).

Selon l'Invention, la chaîne hydrocarbonée de la molécule bifonctionnelle de formule (I) comprend de 2 à 18 atomes de carbone ; une telle longueur de chaîne (inférieure à 20 atomes de carbone) ne permet pas de conduire à un support solide comportant une monocouche autoassemblée organisée (SAM). En effet, selon A. ULMAN, Chem. Rev., 1996, **96**, 1533-1554, la formation de SAM sur des supports solides à l'aide de composés organosiliciés comportant une extrémité tri-fonctionnelle (de type alkyltrichlorosilane) est possible lorsque la chaîne hydrocarbonée contient au moins 20 atomes de carbone.

Au contraire, selon l'invention, l'utilisation de molécules de formule (I), dans lesquelles le groupement A permettant la fixation de celles-ci sur le support est monofonctionnel, permet d'obtenir des couches mono ou sub monomoléculaire denses de façon reproductible. De plus, les molécules de formule (I) qui sont fixées en surface du support ont chacune leur point d'ancrage directement sur la surface, contrairement aux molécules di- ou tri-fonctionnelles qui conduisent souvent à des couches greffées épaisses, rugueuses et mal définies.

Les supports solides pouvant être fonctionnalisés selon ce procédé sont des surfaces organiques (matières plastiques) ou minérales, de préférence poreuses ou planes, de types oxydes métalliques, silice et ses dérivés (verre, quartz, ...), c'est-à-dire toute surface qui présente des fonctions hydroxyle en surface, ainsi que les semi-conducteurs de type silicium présentant des hydrures en surface (Si-H).

Le groupement monofonctionnel A peut notamment être choisi parmi les groupements dialkyl(dialkylamino)silane, dialkylhalogénosilane, diphényl(dialkylamino)silane, diphénylhalogénosilane, [(monoalkyl), (monophényl),(dialkylamino)]-silane, [(monoalkyl),(monophényl),(halogéno)-silane, alcène, alcyne et les composés organométalliques tels que des organomagnésiens ou des organolithiens.

Le groupement protecteur R des molécules de formule (I) ci-dessus peut être choisis parmi les groupements décrits dans *Protective groups in organic synthesis* (T. W. GREENE *et al*., 2^{nd} edition, Wiley Interscience), tels que par exemple un radical alkyle en C₁-C₄ ou un radical cyclique.

Selon une forme avantageuse de ce procédé, et lorsque la surface du support solide présente des fonctions hydroxyle en surface, les molécules de formule (I) sont de préférence choisies parmi les composés organosiliciés de formule (Ia) suivante : 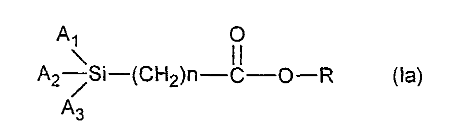 dans laquelle :
- A₁ et A₂, identique ou différents, représentent un radical alkyle en C₁-C₄ ou un groupement phényle,
- A₃ représente un radical alcoxy en C₁-C₄, dialkyl(C₁-C₄)amino ou un atome d'halogène tel que le chlore,
- R représente un groupement protecteur choisi parmi les radicaux alkyle en C₁-C₄ et les radicaux cycliques tel que le radical benzyle,
- n est nombre entier compris entre 2 et 18.

Parmi les radicaux alkyle en C₁-C₄ définis pour A₁ et A₂, on peut notamment citer les radicaux méthyle, éthyle, propyle et butyle, le radical méthyle étant particulièrement préféré.

Parmi les radicaux dialkyl(C₁-C₄)amino définis pour A₃, on peut notamment citer le radical diméthylamino.

Parmi les radicaux alkyle en C₁-C₄ définis pour le groupement R, on peut notamment citer les radicaux méthyle, éthyle, isopropyle et *t*-butyle, le radical *t*-butyle étant particulièrement préféré.

Selon l'invention, les composés de formule (Ia) sont plus particulièrement choisis parmi ceux dans lesquels :
- A₁ et A₂, identiques, représentent un radical méthyle,
- A₃ représente un radical diméthylamino ou un atome de chlore,
- n = 10, et
- R représente un radical *t*-butyle.

Dans le cas particulier où A₃ représente un radical diméthylamino, la molécule correspondante ((CH₃)₂N-Si(CH₃)₂-(CH₂)₁₀-C-(O)-O-*t*-butyle), qui n'est pas commerciale, peut être préparées selon des méthodes classiques d'estérification de l'acide undécylénique (Trost B. M. *et al*., "*Comprehensive organic synthesis*", 1991, **6**, Pergamon Press ; Staab H. A. *et al*., *"Azolides in organic synthesis and biochemistry*", 1998, Wiley Weinheim) et d'hydrosilylation (Fleming I., "*Comprehensive organic synthesis*", volume III, Chapitre 13).

Selon une autre forme avantageuse de ce procédé, et lorsque la surface du support solide présente des fonctions hydrure en surface, les molécules de formule (I) sont de préférence choisies parmi les alcènes, les alcynes, les aldéhydes, les peroxydes ou bien encore les composés organométalliques. Parmi ces composés, les esters méthylique ou *t*-butylique de l'acide undécylénique sont particulièrement préférés.

Lorsque l'étape a) est une étape de silylation, elle peut aussi bien être réalisée en phase organique, aqueuse, catalysée ou non, qu'en phase vapeur.

Lorsque qu'elle est effectuée en phase organique, l'étape a) comprend avantageusement les étapes suivantes :
i) l'élimination des contaminants du support solide et l'hydroxylation de sa surface,
ii) l'introduction dans un solvant choisi parmi les solvants hydrocarbonés non polaires, les solvants polaires et leurs mélanges, sous atmosphère inerte, d'un composé organosilicié de formule générale (Ia) telle que définie ci-dessus,
iii) la silanisation du support obtenu à l'étape i) par immersion dans la solution préparée à l'étape ii) et
iv) le recuit du support silanisé obtenu à l'étape iii), après évaporation du solvant sous atmosphère inerte et à une température comprise entre 50 et 200°C, de préférence à 140°C, pendant une durée comprise entre 2 et 72 heures, et
v) le nettoyage et le séchage du support modifié obtenu à l'étape iv).

Par « contaminants » du support solide, on entend tout composé tel que de la graisse, des poussières ou autres, présent à la surface du support et qui ne fait pas partie de la structure chimique du support lui-même.

Selon la nature du support solide, l'étape i) peut être effectuée à l'aide d'un ou de plusieurs solvants et/ou oxydants et/ou hydroxylants (par exemple un mélange sulfochromique), d'un détergent (par exemple du Hellmanex®), d'un traitement photochimique à l'ozone ou tout autre traitement approprié.

Lors de l'étape ii), la concentration du composé organosilicié de formule (Ia) dans le solvant est de préférence comprise entre 10⁻⁵ et 1 mole/litre.

Lorsque l'étape a) est une étape d'hydrosilylation, et lorsque le support solide utilisé est un support en verre ou en silice, ladite étape a) comprend de préférence les étapes suivantes :
i) l'élimination des contaminants du support solide et l'hydratation de sa surface,
ii) le lavage du support dans un alcool anhydre tel que du méthanol anhydre,
iii) la mise en contact du support avec un alcène désoxygéné au préalable sous argon,
iv) l'hydrosilylation du support par ajout d'un catalyseur (catalyseur de Speier ou de Karstedt, éthyldichloroaluminium) pendant 2 à 24 heures à une température comprise entre 90 et 200°C.

Selon l'invention, l'étape de greffage a) permet de fixer une quantité de molécules de formule (I) comprise entre 1 et 8 µmol/m² de substrat.

De préférence, l'étape de greffage doit laisser intacte la protection de l'acide carboxylique de la molécule bifonctionnelle de formule (I) afin d'assurer un meilleur ordonnancement de la couche greffée, la densité de greffage, ainsi que la reproductibilité de la réaction. Cependant, dans certains cas, il se produit une déprotection des fonctions acide carboxylique lors de certaines réactions d'hydrosilylation ou bien lorsque l'on utilise un chlorosilane à titre de composé de formule (la).

Lorsque l'étape de greffage a) est terminée, il est donc nécessaire de déprotéger les fonctions acides carboxyliques qui n'auraient pas été déprotégées lors de l'étape de greffage.

Selon une première variante du procédé conforme à l'invention, l'étape de déprotection b) peut être réalisée en conditions douces selon une méthode consistant à former un ester de silyle, facilement hydrolysable dans l'eau, de préférence en faisant réagir le support avec un silane de formule (II) suivante :

(alkyl C₁-C₄)₃-Si-Y (II)

dans laquelle Y représente un atome d'halogène tel que l'iode, le brome ou le chlore.

L'utilisation d'une petite molécule de silane de formule (II) présente l'avantage de compléter la couverture du support (passivation) puisque cette petite molécule de silane réagit également avec les fonctions hydroxyles résiduelles du support qui n'ont pas réagi avec les molécules bifonctionnelles de formule (I) au cours de la réaction de greffage ou qui se sont formés par hydroxylation des hydrures sur le silicium pendant l'étape d'hydrosilylation.

La formation de ces esters peut être obtenue :
- soit par réaction du support et d'un iodotrialkylsilane tel que le iodotriméthylsilane dans un solvant organique anhydre (tel que le chloroforme ou le dichlorométhane) à température ambiante ou en chauffant jusqu'à une température de 60°C environ,
- soit par réaction du support et du chlorotriméthylsilane en présence d'iodure de sodium dans de l'acétonitrile ou de l'acétone anhydre à température ambiante ou à 40°C environ.

Dans ce cas l'étape c) de passivation se trouve donc réalisée de façon simultanée par l'étape b).

Selon une deuxième variante du procédé conforme à l'invention, l'étape b) est une déprotection thermique. Cette méthode de déprotection est applicable à certains esters d'acide carboxylique tels que les esters isopropylique et *t*-butylique.

Cette déprotection thermique peut être réalisée par traitement du support fonctionnalisé à une température comprise entre 250 et 280°C pendant 30 à 60 minutes, en atmosphère inerte (argon ou azote par exemple).

Cette étape de déprotection thermique, réalisée après l'étape de silylation ou d'hydrosilylation, permet de générer *in situ*, par simple élévation de la température, la fonction acide carboxylique en surface.

De façon avantageuse, cette étape de déprotection thermique peut donc être réalisée de façon simultanée à l'étape a), par simple élévation de la température à la fin de l'étape a).

Également de façon avantageuse, la déprotection thermique peut être réalisée de façon localisée, par élévation de la température localement, par exemple à l'aide d'un faisceau laser ou de tout autre moyen approprié, de façon à définir des zones activables entourées de zones dans lesquelles les fonctions acide carboxylique restent protégées.

Selon l'invention, la déprotection des fonctions acide carboxylique est de préférence réalisée par formation d'un ester de silyle, puisque cette méthode permet à la fois de générer des fonctions acide carboxyliques en conditions douces et de bloquer les fonctions hydroxyle résiduelles du support (étape de passivation du support), ce qui permet par la suite de limiter l'adsorption non spécifique des molécules sur le support. C'est en effet l'adsorption non spécifique des molécules biologiques qui est responsable du bruit de fond lors de la détection.

Par conséquent, lorsque la déprotection des fonctions acide carboxylique est réalisée de façon thermique, on effectue ensuite l'étape de passivation des fonctions hydroxyle ou hydrure du support en faisant par exemple réagir celui-ci avec un silane de formule (II) tel que décrit ci-dessus ou par toute autre méthode classiquement connue de l'homme de l'art telle que par exemple par réaction du support avec de l'hexaméthyldisilasane ou bien encore selon une méthode d'adsorption forte de petites molécules telles que le méthanol.

Lorsque les étapes de déprotection des fonctions acide carboxylique et de passivation du support sont terminées, les supports fonctionnalisés sont lavés à l'eau et séchés par exemple par un jet d'air comprimé.

L'invention a également pour objet le support solide fonctionnalisé obtenu en mettant en oeuvre le procédé de fonctionnalisation conforme à l'invention et tel que décrit précédemment.

Ces supports fonctionnalisés permettent l'immobilisation reproductible de molécules biologiques d'intérêt tout en limitant l'adsorption non spécifique de molécules et donc en augmentant le rapport signal/bruit lors de la détection. Ils permettent également de limiter les bavures et les auréoles des dépôts localisés par contrôle de l'étalement des gouttes de réactifs.

Ces supports fonctionnalisés peuvent en particulier être utilisés pour l'immobilisation, par fixation covalente, de molécules biologiques d'intérêt porteuses de groupements fonctionnels aminés ou hydroxylés, telles que des acides nucléiques, des polypeptides (protéines, enzymes), des lipides, des carbohydrates ou des hormones.

Au sens de la présente invention et dans ce qui suit, on entend par « acides nucléiques » aussi bien des oligonucléotides que des ADN ou des ARN, ou encore des acides nucléiques au squelette ou aux bases modifiés, tels que les acides nucléiques peptidiques (PNA : Peptide Nucleic Acids) qui font intervenir des liaisons peptidiques à la place des liaisons phosphodiesters.

La présente invention a donc également pour objet un procédé d'immobilisation de molécules biologiques sur support solide fonctionnalisé, caractérisé en ce qu'il comporte les étapes suivantes :
a) la préparation d'un support solide fonctionnalisé comportant des fonctions acide carboxylique terminales sous forme d'ester selon le procédé tel que défini précédemment,
b) la déprotection et l'activation des fonctions acides carboxyliques terminales,
c) la mise en contact du support solide modifié obtenu à l'étape a) ou b) avec une ou plusieurs solutions appliquées localement, dans un ou plusieurs solvants, de la(des) molécules(s) biologique(s) à immobiliser, lesdites molécules biologiques portant à l'une de leurs extrémités une fonction amine ou une fonction hydroxyle ou un bras espaceur fonctionnalisé par une fonction amine primaire,
d) l'évaporation du solvant afin de provoquer la fixation covalente de la(des) molécule(s) biologique(s) au niveau des fonctions acide carboxylique,
e) l'inactivation des fonctions acide carboxylique activées n'ayant pas réagi avec les molécules biologiques à l'aide d'une amine en phase gazeuse ou en solution, et
f) le lavage du support solide sur lequel sont immobilisées lesdites molécules biologiques.

L'étape b) d'activation des fonctions acide carboxylique peut par exemple être effectuée à l'aide d'une solution de N-hydroxysuccinimide, de carbonyldiimidazole ou de carbodiimide, ou encore de tout autre réactif d'activation convenable et connu de l'Homme du métier.

A titre d'exemple, cette étape d'activation peut être réalisée en présence de carbonyldiimidazole (0,1 M) dans du tetrahydrofurane (THF) anhydre, en deux heures environ à température ambiante. Ce type d'activation permet notamment d'obtenir des surfaces activées très réactives vis-à-vis de réactifs nucléophiles (amines, alcools, eau). Dans ce cas, on fait réagir les molécules biologiques dans des solvants anhydres en présence d'une amine tertiaire comme la triéthylamine pour assurer la présence de la forme amino du groupement fonctionnel des molécules biologiques.

Au cours de l'étape c), la concentration de la ou des solutions de molécules biologiques est de préférence comprise entre 10⁻⁶ à 10⁻³ mol/l.

Au cours de l'étape c), il est bien entendu que, pour solubiliser les molécules biologiques, tout solvant aqueux ou anhydre permettant une bonne dissolution de ces dernières et un contrôle ultérieur de l'évaporation de la solution pourrait être utilisé. La nature du solvant devra bien entendu être choisie en fonction de la nature de la molécule biologique à isoler. On peut citer, à titre d'exemple et de façon non limitative les tampons phosphate, le diméthylsulfoxyde (DMSO), l'acétonitrile anhydre ou bien encore un mélange acétonitrile/eau.

Chaque solution de molécules biologiques à immobiliser peut être déposée en un endroit déterminé du support par un moyen adapté de microdéposition.

Ainsi, lorsque la réaction d'immobilisation est réalisée en conditions anhydres, par exemple pour immobiliser des oligonucléotides, il est par exemple possible d'utiliser du DMSO anhydre. La réaction sera, bien entendu, conduite sous atmosphère anhydre. Dans ce cas, les oligonucléotides sont de préférence présents au sein de la solution à une concentration comprise entre 10⁻⁵ et 10⁻³ mol/l.

Les brins d'ADN et les oligonucléotides sont solubles en solution aqueuse en toutes proportions. Par conséquent, lorsque l'on souhaite immobiliser ce type de molécules biologique, l'étape d'immobilisation est de préférence réalisée en condition aqueuse. L'activation des fonctions acide carboxylique par le N-hydroxysuccinimide permet de travailler en condition aqueuse.

Selon l'Invention, l'étape d) d'immobilisation des molécules biologiques, par évaporation du solvant, est cruciale au bon déroulement du procédé d'immobilisation car elle permet le greffage covalent des molécules biologiques d'intérêt sur le support fonctionnalisé. En effet, le procédé d'immobilisation conforme à l'invention met de préférence en oeuvre des solutions très diluées de molécules biologiques (de l'ordre de 10⁻⁶ à 10⁻³ mol/l) dont la détection serait ensuite impossible si le greffage par liaison covalente n'était pas effectué. De façon préférée, cette étape d'évaporation est réalisée lentement, c'est-à-dire pendant un temps compris entre 1 et 10 heures environ.

Lorsque l'étape d'immobilisation est terminée, on procède ensuite à l'inactivation des fonctions acide carboxylique activées n'ayant pas réagi avec les molécules biologiques, à l'aide d'une amine en phase gazeuse ou en solution (étape e)).

Cette étape d'inactivation permet d'éviter les problèmes de bavure des gouttes, de contamination ou de pollution au cours des étapes suivantes, elle peut également être dénommée "capping".

Selon une forme de réalisation avantageuse du procédé d'immobilisation conforme à l'invention, cette étape d'inactivation est réalisée en faisant réagir, en phase gazeuse, de la méthylamine ou de la diméthylamine, préférentiellement de la diméthylamine, pendant 15 à 45 minutes.

Cette étape d'inactivation peut aisément permettre de traiter plusieurs supports de façon simultanée.

Selon une autre forme de réalisation avantageuse de ce procédé, l'étape d'inactivation est réalisée uniquement sur des zones prédéfinies d'un même support, les zones ne devant pas être inactivées étant préalablement protégées par exemple par des gouttes d'eau, de solvants organiques ou d'huile minérale.

La désactivation des zones non protégées permet alors de créer un réseau de zones activées ou réactivables et de zones inertes chimiquement.

Cette forme de réalisation du procédé permet d'aboutir à des supports résolus spatialement qui peuvent par exemple être obtenus par dépôts de gouttes d'eau sur une surface comportant des fonctions acide carboxylique activées (par exemple au carbonyldiimidazole) suivis de l'étape d'inactivation des surfaces accessibles par une amine en phase gazeuse, puis d'un rinçage du support. Les zones inactivées par les gouttes d'eau peuvent ensuite être réactivées avant l'étape de fixation covalente des molécules biologiques.

L'étape finale f) de lavage est destinée à désorber les molécules qui ne seraient pas fixées de façon covalente sur le support fonctionnalisé conforme à l'invention afin d'obtenir une densité reproductible de molécules biologiques fixées en surface.

Cette étape de lavage est de préférence réalisée en soumettant ledit support à des étapes successives de lavages de plus en plus stringents, allant par exemple d'un lavage à l'eau à température d'environ 80°C pendant une heure environ à un lavage dans une solution aqueuse de dodécyl sulfate de sodium (SDS) concentrée, par exemple à 10 %, à une température d'environ 80°C pendant 1 heure environ, suivie éventuellement d'une sonication de quelques minutes.

La réutilisation des supports est conditionnée par l'obtention de signaux reproductibles. Cette étape de lavage assure un rôle en ce qui concerne la réutilisation de ces supports puisqu'elle permet de garantir que les supports ne relargueront pas de molécules au cours des étapes suivantes éventuelles de détection.

Bien que cette étape de lavage ait pour conséquence d'éliminer un certain nombre de molécules biologiques non fixées de façon covalente, et par conséquent de diminuer le taux d'immobilisation, donc l'intensité du signal de détection, elle permet également de diminuer le taux d'adsorption non spécifique et donc le bruit de fond lors de la détection. Le bilan global de cette étape de lavage est donc l'amélioration du rapport signal/bruit ainsi que de la reproductibilité de la mesure.

La présente invention a également pour objet les supports solides obtenus en mettant en oeuvre le procédé d'immobilisation conforme à l'invention, c'est-à-dire les supports solides sur lesquels sont immobilisés par fixation covalente les molécules biologiques d'intérêt.

Ces supports solides peuvent aussi bien être utilisés comme outils d'analyse (diagnostic, séquençage, etc...) que comme outils de préparation (isolation, séparation de molécules complexes) ou de revêtement de surfaces à propriétés spécifiques (revêtements pour chromatographies, revêtements "actifs" tels que des revêtements antistatiques, antibactériens, etc...).

Ils trouvent donc des applications dans de nombreux domaines tels que :
- la synthèse sur supports solides,
- la séparation et la purification de molécules (électrophorèse, chromatographie),
- en biologie moléculaire, en particulier lorsque les molécules biologiques immobilisées sont des enzymes,
- à titre de biocapteurs ("immunoassays", technique d'analyse d'ADN basées sur les "microarrays" par exemple le séquençage par hybridation SBH, single nucleotide polymorphism (SNP) et le diagnostic biomédical).

L'utilisation de supports solides fonctionnalisés selon la présente invention permet d'immobiliser différents types de molécules biologiques et donc de préparer différents types de puces comme des puces à acides nucléiques telles que des puces à ADN, des puces à polypeptides telles que des puces à protéines, etc...

L'utilisation de supports solides modifiés selon la présente invention est particulièrement avantageuse pour la préparation de puces à ADN, à savoir de supports sur lesquels sont fixés, de façon covalente, un ensemble d'ADN de séquences connues dans un ordre bien précis, ces puces étant réutilisables de nombreuses fois. De telles puces à ADN permettent, par hybridation des ADN immobilisés sur le support avec des acides nucléiques ou des oligonucléotides cibles, de déterminer la séquence de ces molécules cibles ou de suivre l'expression de gènes. Les applications sont nombreuses : découverte de nouveaux gènes, de nouveaux médicaments, réalisation de diagnostics, études de toxicité, etc.

La présente invention a donc, en outre, pour objet une puce à acides nucléiques ou à polypeptides, caractérisée en ce qu'elle est obtenue par le procédé d'immobilisation de molécules biologiques selon la présente invention, dans lequel lesdites molécules biologiques sont des acides nucléiques ou des polypeptides.

Les puces à acides nucléiques ou à polypeptides selon l'invention présentent l'avantage d'être stables et donc de pouvoir être réutilisées de nombreuses fois, en particulier les puces à ADN peuvent être réutilisées dans de nombreux cycles d'hybridation et de dénaturation.

Outre les dispositions qui précèdent, l'invention comprend encore d'autres dispositions qui ressortiront de la description qui va suivre, qui se réfère à un exemple de fonctionnalisation de support solide, à un exemple d'immobilisation d'oligonucléotides ainsi qu'à un exemple mettant en évidence la reproductibilité du procédé d'immobilisation conforme à l'invention.

Il doit être bien entendu, toutefois, que ces exemples sont donnés uniquement à titre d'illustration de l'objet de l'invention, dont ils ne constituent en aucune manière une limitation.

### Exemple 1 : Préparation d'un support solide fonctionnalisé

### 1) Nettoyage et hydroxylation des échantillons

Des lames de verre (76 x 26 mm) sont immergées dans une solution d'acide sulfurique et d'eau oxygénée (7/3 v/v) à 80°C pendant une heure. Les lames sont ensuite rincées abondamment à l'eau ultra pure puis séchées par une soufflette de gaz. Elles sont enfin placées dans un réacteur en verre.

### 2) Silanisation

Le réacteur contenant les lames de verre est chauffé à 140°C pendant une heure sous vide puis pendant une heure sous un balayage de gaz inerte sec (argon, azote). Le réacteur est ensuite refroidi dans un bain d'eau glacée et 150 ml de pentane sont injectés de façon à recouvrir la surface des échantillons. On injecte ensuite 300 µl de silane.

Le pentane est ensuite évaporé sous vide, puis le réacteur est chauffé à 140°C et balayé par un courant de gaz inerte (argon ou azote) pendant 12 heures.

### 3) Rincage des échantillons

En fin de silanisation, les lames de verre sont lavées dans du tetrahydrofuranne (THF) sous ultrasons, avant d'être stockées ou de subir les autres étapes de la fonctionnalisation.

### EXEMPLE 2 : Immobilisation d'oligonucléotides sur un support fonctionnalisé conforme à l'invention

### 1) Déprotection des fonctions acide carboxylique

Les lames de verre fonctionnalisées obtenues ci-dessus à l'exemple 1 sont séchées sous atmosphère inerte à 140°C pendant 2 heures. Après refroidissement, elles sont immergées dans une solution de dichlorométhane (DCM) préalablement séché sur alumine, puis du iodotriméthylsilane est injecté en quantité suffisante pour atteindre une concentration de 0,1M. L'hydrolyse est conduite pendant une nuit à température ambiante. En fin de réaction, les lames sont lavées avec de l'eau désionisée et séchées par un jet d'air comprimé.

### 2) Activation des échantillons au N-hydroxysuccinimide

Les lames sont séchées à 140°C sous argon pendant deux heures. Après refroidissement, elles sont immergées dans une solution de THF anhydre contenant du N-hydroxysuccinimide (0,1 M) et du diisopropylcarbodiimide (0,1 M). L'activation est conduite pendant 5 heures à température ambiante. Les lames sont ensuite rincées au THF anhydre puis stockées sous atmosphère inerte à l'obscurité.

### 3) Immobilisation des oligonucléotides

Les oligonucléotides (25 mères, porteurs d'un bras espaceur fonctionnalisé par une fonction amine (C₆-NH₂)), sont solubilisés dans du tampon phosphate PBS (Phosphate-Buffered Saline) de pH 8,5. Des surfaces de 1 cm² sont recouvertes de 50 µl de solution d'oligonucléotides. Après évaporation lente du tampon PBS à une température de 50°C, les lames sont inactivées par blocage (capping) des fonctions acides qui n'auraient pas réagi avec les fonctions amine des bras espaceur des oligonucléotides. Pour ce faire, les lames sont traitées par la méthylamine en phase gazeuse pendant 30 minutes.

### 4) Lavages des lames

Pour finir, les surfaces sont lavées dans des bains successifs de plus en plus stringents : eau ultra pure à 100°C pendant 45 minutes, puis SDS à 10 % à 80°C pendant 45 minutes, et enfin SDS à 10 % sous ultrasons pendant 5 minutes.

### EXEMPLE 3 : Mise en évidence de la reproductibilité du procédé d'immobilisation conforme à l'invention

Différentes lames de verres fonctionnalisées selon le procédé décrit ci-dessus à l'exemple 1 et activées au N-hydroxysuccinimide selon le procédé décrit ci-dessus à l'exemple 2 ont été utilisées pour immobiliser 1 µl de solutions de d'oligonucléotides à 12 µmol/l (25 mères, porteurs en position 3' d'un bras espaceur comportant une fonction amine primaire).

Après lavage et hybridation de la séquence complémentaire, les résultats concernant le nombre de brins hybridés par cm² sur trois lames différentes, après un lavage au tampon SSC 1X (Saline Sodium Citrate) qui est une dilution au 1/20 d'un tampon SSC 20X contenant un mélange de NaCl (3 M) et de citrate de sodium (3 M), à une température de 47°C pendant 45 minutes, étaient les suivants (données quantitatives obtenues par marquage radioactif au ³²P) :
- lame n°1 : 2,20.10¹⁰
- lame n° 2 : 2,26.10¹⁰
- lame n° 3 : 2,23.10¹⁰

Ces résultats mettent en évidence la grande reproductibilité du procédé d'immobilisation conforme à l'invention.

Sur une autre lame de verre, une première hybridation avec un double brin PCR de 60 paires de bases a donné une hybridation de l'ordre de 3,95.10⁸ brins/cm². Après dénaturation et réhybridation dans des conditions identiques aux trois lames précédentes, une densité de 2,6 10¹⁰ brins d'oligonucléotides/cm² a été obtenue.

L'ensemble de ces résultats démontre par conséquent la grande reproductibilité des lames entre les lots ainsi que la possibilité de réutiliser les supports sans perte de signal.

## Revendications

1. Procédé de fonctionnalisation d'un support solide comportant en surface des fonctions hydroxyle ou hydrure, comprenant les étapes suivantes :
a) le greffage par fixation covalente d'au moins une molécule bifonctionnelle comportant une fonction acide carboxylique protégée de formule (I) suivante :
A-X-COOR (I)
dans laquelle :
- A représente un groupement permettant la fixation covalente de la molécule bifonctionnelle de formule (I) sur les fonctions hydroxyle ou hydrure du support,
- R représente un groupement protecteur de la fonction acide carboxylique,
- X représente une chaîne hydrocarbonée en C₂-C₁₈, linéaire ou ramifiée, saturée ou insaturée, éventuellement interrompue par un ou plusieurs hétéroatomes choisis parmi N, O et S ;
b) la déprotection des fonctions acide carboxylique n'ayant pas été déprotégées au cours de l'étape a) de greffage ;
**caractérisé en ce que** l'étape a) est réalisée à une température comprise entre 50 et 200°C avec un composé de formule (I) dans lequel A est un groupement monofonctionnel et **en ce que** ledit procédé comprend en outre une étape c) de passivation des fonctions hydroxyle ou hydrure résiduelles du support n'ayant pas réagi avec ladite ou lesdites molécules de formule (I).

2. Procédé selon la revendication 1, **caractérisé par le fait que** les supports solides sont choisis parmi les matières plastiques, les oxydes métalliques, la silice et ses dérivés et les semi-conducteurs.

3. Procédé selon la revendication 1 ou 2, **caractérisé par le fait que** le groupement monofonctionnel A est choisi parmi les groupements dialkyl(dialkylamino)silane, dialkylhalogénosilane, diphényl(dialkylamino)silane, diphénylhalogénosilane, [(monoalkyl),(monophényl),(dialkylamino)]-silane, [(monoalkyl),(monophényl),(halogéno)-silane, alcène, alcyne et les composés organométalliques.

4. Procédé selon l'une quelconque des revendications précédentes, **caractérisé par le fait que** le groupement protecteur R est un radical alkyle en C₁-C₄ ou un radical cyclique.

5. Procédé selon l'une quelconque des revendications précédentes, **caractérisé par le fait que** la surface du support solide présente des fonctions hydroxyle en surface et que les molécules de formule (I) sont choisies parmi les composés organosiliciés de formule (Ia) suivante : 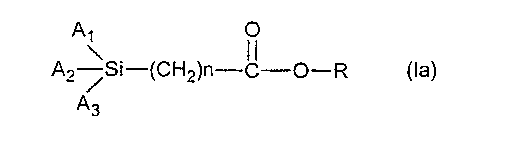 dans laquelle :
- A₁ et A₂, identique ou différents, représentent un radical alkyle en C₁-C₄ ou phényle,
- A₃ représente un radical alcoxy en C₁-C₄, dialkyl(C₁-C₄)amino ou un atome d'halogène tel que le chlore,
- R représente un groupement protecteur choisi parmi les radicaux alkyle en C₁-C₄ et les radicaux cycliques,
- n est nombre entier compris entre 2 et 18.

6. Procédé selon la revendication 5, **caractérisé par le fait que** les composés de formule (Ia) sont choisis parmi ceux dans lesquels :
- A₁ et A₂, identiques, représentent un radical méthyle,
- A₃ représente un radical diméthylamino ou un atome de chlore,
- n = 10, et
- R représente un radical *t*-butyle.

7. Procédé selon l'une quelconque des revendications 1 à 4, **caractérisé par le fait que** la surface du support solide présente des fonctions hydrure en surface et que les molécules de formule (I) sont choisies parmi les alcènes, les alcynes, les aldéhydes, les peroxydes ou bien encore les composés organométalliques.

8. Procédé selon la revendication 7, **caractérisé par le fait que** les composés de formule (I) sont choisis parmi les esters méthylique ou *t*-butylique de l'acide undécylénique.

9. Procédé selon l'une quelconque des revendications 1 à 6, **caractérisé par le fait que** l'étape a) est une étape de silanisation en phase organique comprenant les étapes suivantes :
i) l'élimination des contaminants du support solide et l'hydroxylation de sa surface,
ii) l'introduction dans un solvant choisi parmi les solvants hydrocarbonés non polaires, les solvants polaires et leurs mélanges, sous atmosphère inerte, d'un composé organosilicié de formule générale (Ia) telle que définie ci-dessus,
iii) la silanisation du support obtenu à l'étape i) par immersion dans la solution préparée à l'étape ii) et
iv) le recuit du support silanisé obtenu à l'étape iii), après évaporation du solvant sous atmosphère inerte et à une température comprise entre 50 et 200°C, de préférence à 140°C, pendant une durée comprise entre 2 et 72 heures, et
v) le nettoyage et le séchage du support modifié obtenu à l'étape iv).

10. Procédé selon l'une quelconque des revendications 1 à 4, 7 ou 8, **caractérisé par le fait que** le support solide est un support en verre ou en silice et que l'étape a) est une étape d'hydrosilylation, comprenant les étapes suivantes :
i) l'élimination des contaminants du support solide et l'hydratation de sa surface,
ii) le lavage du support dans un alcool anhydre tel que du méthanol anhydre,
iii) la mise en contact du support avec un alcène désoxygéné au préalable sous argon,
iv) l'hydrosilylation du support par ajout d'un catalyseur pendant 2 à 24 heures à une température comprise entre 90 et 200°C.

11. Procédé selon l'une quelconque des revendications précédentes, **caractérisé par le fait que** l'étape de déprotection b) est réalisée par formation d'un ester de silyle en faisant réagir le support avec un silane de formule (II) suivante :
(alkyl C₁-C₄)₃-Si-Y (II)
dans laquelle Y représente un atome d'halogène tel que l'iode, le brome ou le chlore.

12. Procédé selon la revendication 11, **caractérisé par le fait que** la formation de l'ester de silyle est obtenue :
- soit par réaction du support et d'un iodotrialkylsilane dans un solvant organique anhydre à température ambiante ou en chauffant jusqu'à une température de 60°C environ,
- soit par réaction du support et du chlorotriméthylsilane en présence d'iodure de sodium dans de l'acétonitrile ou de l'acétone anhydre à température ambiante ou à 40°C environ.

13. Procédé selon la revendication 11 ou 12, **caractérisé par le fait que** l'étape c) est réalisée de façon simultanée par l'étape b).

14. Procédé selon l'une quelconque des revendications 1 à 10, **caractérisé par le fait que** l'étape b) est une étape de déprotection thermique.

15. Procédé selon la revendication 14, **caractérisé par le fait que** l'étape de déprotection thermique est réalisée de façon simultanée à l'étape a), par simple élévation de la température à la fin de l'étape a).

16. Support solide fonctionnalisé obtenu selon l'une quelconque des revendications 1 à 15.

17. Utilisation d'au moins un support solide fonctionnalisé tel que défini à la revendication 16, pour l'immobilisation, par fixation covalente, de molécules biologiques d'intérêt porteuses de groupements fonctionnels aminés ou hydroxylés.

18. Utilisation selon la revendication 17, **caractérisée par le fait que** les molécules biologiques sont choisies parmi les acides nucléiques, les polypeptides, les lipides, les carbohydrates et les hormones.

19. Procédé d'immobilisation de molécules biologiques sur un support solide fonctionnalisé, **caractérisé en ce qu'**il comporte les étapes suivantes :
a) la préparation d'un support solide fonctionnalisé comportant des fonctions acide carboxylique terminales sous forme d'ester selon le procédé tel que défini à l'une quelconque des revendications 1 à 15,
b) la déprotection et l'activation des fonctions acides carboxyliques terminales,
c) la mise en contact du support solide modifié obtenu à l'étape a) ou b) avec une ou plusieurs solutions appliquées localement, dans un ou plusieurs solvants, de la(des) molécules(s) biologique(s) à immobiliser, lesdites molécules biologiques portant à l'une de leurs extrémités une fonction amine ou une fonction hydroxyle ou un bras espaceur fonctionnalisé par une fonction amine primaire,
d) l'évaporation du solvant afin de provoquer la fixation covalente de la(des) molécule(s) biologique(s) au niveau des fonctions acide carboxylique,
e) l'inactivation des fonctions acide carboxylique activées n'ayant pas réagi avec les molécules biologiques à l'aide d'une amine en phase gazeuse ou en solution, et
f) le lavage du support solide sur lequel sont immobilisées lesdites molécules biologiques.

20. Procédé selon la revendication 19, **caractérisé par le fait que** l'amine utilisée lors de l'étape e) est choisie parmi la méthylamine et la diméthylamine.

21. Procédé selon la revendication 19 ou 20, **caractérisé par le fait que** l'étape e) est réalisée uniquement sur des zones prédéfinies d'un même support, les zones ne devant pas être inactivées étant préalablement protégées.

22. Support solide obtenu selon le procédé d'immobilisation tel que défini à l'une quelconque des revendications 19 à 21.

23. Support solide selon la revendication 22, **caractérisé par le fait que** les molécules biologiques immobilisées sont des acides nucléiques ou des polypeptides et qu'il constitue une puce à acides nucléique ou à polypeptides.

24. Utilisation d'un support solide tel que défini à la revendication 22 ou 23, pour
- la synthèse sur supports solides,
- la séparation et la purification de molécules,
- en biologie moléculaire, en particulier lorsque les molécules biologiques immobilisées sont des enzymes, ou encore
- à titre de biocapteurs.

## Patentansprüche

1. Verfahren zur Funktionalisierung eines festen Trägers, der an der Oberfläche Hydroxyl- oder Hydridfunktionen trägt, umfassend die folgenden Schritte:
a) Pfropfen mindestens eines bifunktionellen Moleküls mittels kovalenter Bindung, das eine geschützte Carbonsäurefunktion der folgenden Formel (I) trägt:
A-X-COOR (I)
worin:
- A eine Gruppe darstellt, die eine kovalente Bindung des bifunktionellen Moleküls der Formel (I) an die Hydroxyl- oder Hydridfunktionen des Trägers gestattet,
- R eine Schutzgruppe der Carbonsäurefunktion darstellt,
- X eine gerade oder verzweigte, gesättigte oder ungesättigte C₂-C₁₈-Kohlenwasserstoffkette darstellt, die gegebenenfalls durch ein oder mehrere Heteroatome, die aus N, O und S ausgewählt sind, unterbrochen ist;
b) Entfernen der Schutzgruppen von den Carbonsäurefunktionen, von denen im Verlauf des Pfropfungsschrittes a) nicht die Schutzgruppe entfernt wurde;
**dadurch gekennzeichnet, dass** der Schritt a) bei einer Temperatur zwischen 50 und 200°C mit einer Verbindung der Formel (I) durchgeführt wird, in der A eine monofunktionelle Gruppe ist, und **dadurch**, dass das Verfahren ferner einen Schritt c) der Passivierung der restlichen Hydroxyl- oder Hydridfunktionen des Trägers, die nicht mit dem oder den Molekül(en) der Formel (I) umgesetzt wurden, umfasst.

2. Verfahren nach Anspruch 1, **dadurch gekennzeichnet, dass** die festen Träger aus Kunststoffmaterialien, Metalloxiden, Siliciumoxid und seinen Derivaten und Halbleitern ausgewählt sind.

3. Verfahren nach Anspruch 1 oder 2, **dadurch gekennzeichnet, dass** die monofunktionelle Gruppe A aus Dialkyl(dialkylamino)silan-, Dialkylhalogensilan-, Diphenyl(dialkylamino)silan-, Diphenylhalogensilan-, [(Monoalkyl),(Monophenyl),(Dialkylamino)]silan-, [(Monoalkyl),(Monophenyl),(Halogen)]silan-, Alken-, Alkingruppen und metallorganischen Verbindungen ausgewählt ist.

4. Verfahren nach einem der vorstehenden Ansprüche, **dadurch gekennzeichnet, dass** die Schutzgruppe R ein C₁-C₄-Alkylrest oder ein zyklischer Rest ist.

5. Verfahren nach einem der vorstehenden Ansprüche, **dadurch gekennzeichnet, dass** die Oberfläche des festen Trägers oberflächenständige Hydroxylfunktionen besitzt und dass die Moleküle der Formel (I) aus den Organosiliciumverbindungen der folgenden Formel (la) ausgewählt sind: 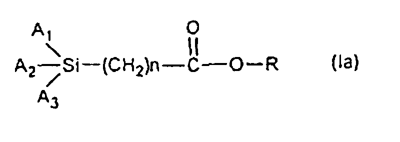 worin:
- A₁ und A₂ gleich oder verschieden sind und einen C₁-C₄-Alkyl- oder einen Phenylrest darstellen,
- A₃ einen C₁-C₄-Alkoxy-, Dialkyl-(C₁-C₄)amino-Rest oder ein Halogenatom, wie Chlor, darstellt,
- R eine Schutzgruppe darstellt, die aus C₁-C₄-Alkylresten und zyklischen Resten ausgewählt ist,
- n eine ganze Zahl zwischen 2 und 18 ist.

6. Verfahren nach Anspruch 5, **dadurch gekennzeichnet, dass** die Verbindungen der Formel (Ia) aus denjenigen ausgewählt sind, in denen:
- A₁ und A₂ gleich sind und einen Methylrest darstellen,
- A₃ einen Dimethylaminorest oder ein Chloratom darstellt,
- n = 10 ist und
- R einen *t*-Butylrest darstellt.

7. Verfahren nach einem der Ansprüche 1 bis 4, **dadurch gekennzeichnet, dass** die Oberfläche des festen Trägers oberflächenständige Hydridfunktionen besitzt und dass die Moleküle der Formel (I) aus Alkenen, Alkinen, Aldehyden, Peroxiden oder aus metallorganischen Verbindungen ausgewählt sind.

8. Verfahren nach Anspruch 7, **dadurch gekennzeichnet, dass** die Verbindungen der Formel (1) aus Methyl- oder *t*-Butylestern der Undecylensäure ausgewählt sind.

9. Verfahren nach einem der Ansprüche 1 bis 6, **dadurch gekennzeichnet, dass** der Schritt a) ein Schritt der Silanisierung in organischer Phase ist, der die folgenden Schritte umfasst:
i) Beseitigung von Verunreinigungen des festen Trägers und Hydroxylierung seiner Oberfläche,
ii) Einbringen einer Organosiliciumverbindung der allgemeinen Formel (Ia), wie vorstehend definiert, in ein Lösungsmittel, das aus unpolaren Kohlenwasserstofflösungsmitteln, polaren Lösungsmitteln und ihren Gemischen ausgewählt ist, unter einer inerten Atmosphäre,
iii) Silanisierung des im Schritt i) erhaltenen Trägers mittels Eintauchen in die im Schritt ii) hergestellte Lösung und
iv) Härten des im Schritt iii) erhaltenen silanisierten Trägers nach Verdampfen des Lösungsmittels unter einer inerten Atmosphäre und bei einer Temperatur zwischen 50 und 200°C, vorzugsweise bei 140°C, für eine Dauer zwischen 2 und 72 Stunden und
v) Waschen und Trocknen des im Schritt iv) erhaltenen modifizierten Trägers.

10. Verfahren nach einem der Ansprüche 1 bis 4, 7 oder 8, **dadurch gekennzeichnet, dass** der feste Träger ein Träger aus Glas oder Siliciumoxid ist und dass der Schritt a) ein Hydroxysilylierungsschritt ist, der die folgenden Schritte umfasst:
i) Beseitigung von Verunreinigungen des festen Trägers und Hydratisierung seiner Oberfläche,
ii) Waschen des Trägers in einem wasserfreien Alkohol, wie wasserfreiem Methanol,
iii) In-Kontakt-bringen des Trägers mit einem Alkohol, der zuvor unter Argon desoxygeniert wurde,
iv) Hydroxysilylierung des Trägers durch Zugabe eines Katalysators während 2 bis 24 Stunden bei einer Temperatur zwischen 90 und 200°C.

11. Verfahren nach einem der vorstehenden Ansprüche, **dadurch gekennzeichnet, dass** der Schritt der Entfernung der Schutzgruppe b) mittels Bildung eines Silylesters durchgeführt wird, indem der Träger mit einem Silan der folgenden Formel (II) umgesetzt wird:
(C₁-C₄-Alkyl)₃-Si-Y (II)
worin Y ein Halogenatom, wie Iod, Brom oder Chlor, darstellt.

12. Verfahren nach Anspruch 11, **dadurch gekennzeichnet, dass** die Bildung des Silylesters
- entweder durch Umsetzung des Trägers und eines Iodtrialkylsilans in einem wasserfreien organischen Lösungsmittels bei Umgebungstemperatur oder unter Erhitzen bis auf eine Temperatur von etwa 60°C
- oder durch Umsetzung des Trägers mit Chlortrimethylsilan in Gegenwart von Natriumiodid in wasserfreiem Acetonitril oder Aceton bei Umgebungstemperatur oder etwa 40°C
erhalten wird.

13. Verfahren nach Anspruch 11 oder 12, **dadurch gekennzeichnet, dass** der Schritt c) gleichzeitig mit Schritt b) durchgeführt wird.

14. Verfahren nach einem der Ansprüche 1 bis 10, **dadurch gekennzeichnet, dass** der Schritt b) ein thermischer Schutzgruppenentfernungsschritt ist.

15. Verfahren nach Anspruch 14, **dadurch gekennzeichnet, dass** der thermische Schutzgruppenentfemungsschritt gleichzeitig mit Schritt a) durch einfache Erhöhung der Temperatur am Ende von Schritt a) durchgeführt wird.

16. Funktionalisierter fester Träger, der gemäß einem der Ansprüche 1 bis 15 erhalten wird.

17. Verwendung mindestens eines funktionalisierten festen Trägers, wie im Anspruch 16 definiert, zur Immobilisierung biologischer Moleküle von Interesse, die aminierte oder hydroxylierte funktionelle Gruppen tragen, durch kovalente Bindung.

18. Verwendung nach Anspruch 17, **dadurch gekennzeichnet, dass** die biologischen Moleküle aus Nucleinsäuren, Polypeptiden, Lipiden, Kohlenhydraten und Hormonen ausgewählt sind.

19. Verfahren zur Immobilisierung biologischer Moleküle auf einem funktionalisierten festen Träger, **dadurch gekennzeichnet, dass** es die folgenden Schritte umfasst:
a) Herstellung eines funktionalisierten festen Trägers, der endständige Carbonsäurefunktionen in Esterform trägt, gemäß dem Verfahren, wie in einem der Ansprüche I bis 15 definiert,
b) Entfernen der Schutzgruppen und Aktivierung der endständigen Carbonsäurefunktionen,
c) In-Kontakt-bringen des im Schritt a) oder b) erhaltenen modifizierten festen Trägers mit einer oder mehreren lokal aufgebrachten Lösungen in einem oder mehreren Lösungsmitteln, dem (den) zu immobilisierenden biologischen Molekül(en), wobei die biologischen Moleküle an einem ihrer Enden eine Aminfunktion oder eine Hydroxylfunktion oder einen durch eine primäre Aminfunktion funktionalisierten Spacerarm tragen,
d) Verdampfen des Lösungsmittels, um die kovalente Bindung des (der) biologischen Molekül(s/e) an den Carbonsäurefunktionen zu bewirken,
e) Inaktivierung der aktivierten Carbonsäurefunktionen, die nicht mit den biologischen Molekülen umgesetzt wurden, mithilfe eines Amins in der Gasphase oder in Lösung und
f) Waschen des festen Trägers, an dem die biologischen Moleküle immobilisiert sind.

20. Verfahren nach Anspruch 19, **dadurch gekennzeichnet, dass** das im Schritt e) verwendete Amin aus Methylamin und Dimethylamin ausgewählt ist.

21. Verfahren nach Anspruch 19 oder 20, **dadurch gekennzeichnet, dass** der Schritt e) nur in vorbestimmten Zonen eines gleichen Trägers durchgeführt wird, wobei die Zonen, die nicht inaktiviert werden sollen, zuvor geschützt werden.

22. Fester Träger, der gemäß dem Verfahren zur Immobilisierung, wie in einem der Ansprüche 19 bis 21 definiert, erhalten wird.

23. Fester Träger nach Anspruch 22, **dadurch gekennzeichnet, dass** die immobilisierten biologischen Moleküle Nucleinsäuren oder Polypeptide sind, und **dadurch**, dass er einen Nucleinsäurechip oder einen Polypeptidchip bildet.

24. Verwendung eines festen Trägers, wie im Anspruch 22 oder 23 definiert, zur
- Synthese auf festen Trägem,
- Trennung und Reinigung von Molekülen,
- in der Molekularbiologie, insbesondere wenn die immobilisierten biologischen Moleküle Enzyme sind, oder auch
- als biologische Fänger.

## Claims

1. Method of functionalising a solid support comprising hydroxyl or hydride functions on the surface, comprising the following steps:
a) the grafting, by covalent fixing, of at least one bifunctional molecule comprising a protected carboxylic acid function of the following formula (I):
A-X-COOR (I)
in which:
- A represents a group allowing the covalent fixing of the bifunctional molecule of formula (I) on the hydroxyl or hydride functions of the support,
- R represents a group protecting the carboxylic acid function,
- X represents a linear or branched, saturated or unsaturated, C₂-C₁₈ hydrocarbon chain, possibly interrupted by one or more heteroatoms chosen from amongst N, O and S;
b) the deprotection of the carboxylic acid functions that were not deprotected during the grafting step a);
**characterised in that** step a) is performed at a temperature between 50° and 200°C with a compound of formula (I) in which A is a monofunctional group and **in that** the said method also comprises a step c) of passivation of the residual hydroxyl or hydride functions of the support that have not reacted with the said molecule or molecules of formula (I).

2. Method according to claim 1, **characterised by** the fact that the solid supports are chosen from amongst plastics materials, metallic oxides, silica and derivatives thereof and semiconductors.

3. Method according to claim 1 or 2, **characterised by** the fact that the monofunctional group A is chosen from amongst dialkyl(dialkylamino)silane, dialkylhalogenosilane, diphenyl(dialkylamino)silane, diphenylhalogenosilane, [(monoalkyl),(monophenyl),(dialkylamino)]-silane, [(monoalkyl),(monophenyl),(halogeno)]-silane, alkene and alkyne groups and the organometallic compounds.

4. Method according to any one of the preceding claims, **characterised by** the fact that the protecting group R is a C₁-C₄ alkyl radical or a cyclic radical.

5. Method according to any one of the preceding claims, **characterised by** the fact that the surface of the solid support has hydroxyl functions on the surface and the molecules of formula (I) are chosen from amongst the organosilicone compounds of the following formula (Ia) 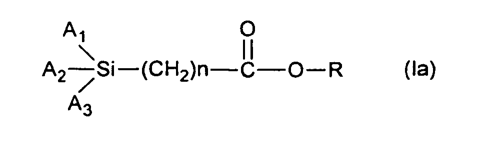 in which:
- A₁ and A₂, identical or different, represent a C₁-C₄ alkyl radical or a phenyl radical,
- A₃ represents a C₁-C₄ alkoxy radical, a dialkyl(C₁-C₄)amino radical or a halogen atom such as chlorine,
- R represents a protecting group chosen from amongst C₁-C₄ alkyl radicals and cyclic radicals,
- n is an integer number comprised between 2 and 18.

6. Method according to claim 5, **characterised by** the fact that the compounds of formula (Ia) are chosen from amongst those in which:
- A₁ and A₂, identical, represent a methyl radical,
- A₃ represents a dimethylamino radical or a chlorine atom,
- n = 10, and
- R represents a t-butyl radical.

7. Method according to any one of claims 1 to 4, **characterised by** the fact that the surface of the solid support has surface hydride functions and that the molecules of formula (I) are chosen from amongst alkenes, alkynes, aldehydes, peroxides or organometallic compounds.

8. Method according to claim 7, **characterised by** the fact that the compounds of formula (I) are chosen from amongst the methyl or t-butyl esters of undecylenic acid.

9. Method according to any one of claims 1 to 6, **characterised by** the fact that step a) is a step of organic phase silanisation comprising the following steps:
i) the elimination of the contaminants from the solid support and the hydroxylation of its surface,
ii) the introduction, into a solvent chosen from amongst nonpolar hydrocarbon solvents, polar solvents and mixtures thereof, under an inert atmosphere, of an organosilicon compound of general formula (Ia) as defined above,
iii) the silanisation of the support obtained at step i) by immersion in the solution prepared at step ii), and
iv) the toughening of the silanised support obtained at step iii), after evaporation of the solvent under an inert atmosphere and at a temperature of between 50° and 200°C, preferably at 140°C, for a period of between 2 and 72 hours, and
v) the cleaning and drying of the modified support obtained at step iv).

10. Method according to any one of claims 1 to 4, 7 or 8, **characterised by** the fact that the solid support is a glass or silica support and in that step a) is a step of hydrosilylation, comprising the following steps:
i) the elimination of the contaminants from the solid support and the hydration of its surface,
ii) the washing of the support in an anhydrous alcohol such as anhydrous methanol,
iii) putting the support in contact with an alkene previously deoxygenated under argon,
iv) the hydrosilylation of the support by the addition of a catalyst for 2 to 24 hours at a temperature between 90° and 200°C.

11. Method according to any one of the preceding claims, **characterised by** the fact that the deprotection step b) is performed by the formation of a silyl ester by making the support react with a silane of the following formula II):
(alkyl C₁-C₄)₃-Si-Y (II)
in which Y represents a halogen atom such as iodine, bromine or chlorine.

12. Method according to claim 11, **characterised by** the fact that the formation of the silyl ester is obtained:
- either by reaction of the support with an iodotrialkylsilane in an anhydrous organic solvent at room temperature or by heating up to a temperature of approximately 60°C,
- or by reaction of the support with chlorotrimethylsilane in the presence of sodium iodide in acetonitrile or anhydrous acetone at room temperature or at approximately 40°C.

13. Method according to claim 11 or 12, **characterised by** the fact that step c) is performed simultaneously by step b).

14. Method according to any one of claims 1 to 10, **characterised by** the fact that step b) is a thermal deprotection step.

15. Method according to claim 14, **characterised by** the fact that the thermal deprotection step is performed simultaneously with step a), by simply raising the temperature at the end of step a).

16. Functionalised solid support obtained according to any one of claims 1 to 15.

17. Use of at least one functionalised solid support as defined in claim 16 for immobilisation, by covalent fixation, of biological molecules of interest carrying amine or hydroxyl functional groups.

18. Use according to claim 17, **characterised by** the fact that the biological molecules are chosen from amongst nucleic acids, polypeptides, lipids, carbohydrates and hormones.

19. Method of immobilising biological molecules on a functionalised solid support, **characterised in that** it comprises the following steps:
a) the preparation of a functionalised solid support comprising terminal carboxylic acid functions in the form of ester according to the method as defined in any one of claims 1 to 15,
b) the deprotection and activation of the terminal carboxylic acid functions,
c) putting the modified solid support obtained at step a) or b) in contact with one or more locally applied solutions, in one or more solvents, of the biological molecule or molecules to be immobilised, the said biological molecules carrying at one of their ends an amine function or a hydroxyl function or a spacer arm functionalised by a primary amine function,
d) the evaporation of the solvent in order to cause the covalent fixation of the biological molecule or molecules at the level of the carboxylic acid functions,
e) the inactivation of the activated carboxylic acid functions that have not reacted with the biological molecules by means of an amine in gaseous phase or in solution, and
f) the washing of the solid support on which the said biological molecules are immobilised.

20. Method according to claim 19, **characterised by** the fact that the amine used at step e) is chosen from amongst methylamine and dimethylamine.

21. Method according to claim 19 or 20, **characterised by** the fact that step e) is performed solely on predefined areas of one and the same support, the areas not having to be inactivated being previously protected.

22. Solid support obtained according to the immobilisation method as defined in any one of claims 19 to 21.

23. Solid support according to claim 22, **characterised by** the fact that the immobilised biological molecules are nucleic acids or polypeptides and in that it constitutes a nucleic acid chip or a polypeptide chip.

24. Use of a solid support as defined in claim 22 or 23, for
- synthesis on solid supports,
- separation and purification of molecules,
- in molecular biology, in particular when the biological molecules immobilised are enzymes, or
- by way of biosensors.
